# EUROPÄISCHE PATENTANMELDUNG

(11) **EP 1 473 308 A1**
(43) Veröffentlichungstag der Anmeldung: **03.11.2004**
(21) Anmeldenummer: 03009523.6
(22) Anmeldetag: 28.04.2003
(51) Int. Cl.: C08B 31/04, C08B 31/08, C08B 31/18, A61K 31/00

(54) **Stärkederivate zur klinischen, insbesondere parenteralen Anwendung**

(71) Anmelder: B. BRAUN MELSUNGEN AG, 34212 Melsungen (DE)
(72) Erfinder: Böttger, Frank, 88212 Ravensburg (DE)
(74) Vertreter: Weber, Thomas, Dr.

(57) **Zusammenfassung**

Die vorliegende Erfindung betrifft physiologisch verträgliche Stärkederivate, ein Verfahren zu deren Herstellung, eine pharmazeutische Zusammensetzung umfassend die erfindungsgemäßen Stärkederivate sowie deren Verwendung zur klinischen, insbesondere parenteralen Anwendung zur Blutplasmasubstitution; Hämodilution sowie zur Verbesserung der Makround Mikrozirkulation.

## Beschreibung

### Gegenstand der Erfindung

Die vorliegende Erfindung betrifft physiologisch verträgliche Stärkederivate, ein Verfahren zu deren Herstellung, eine pharmazeutische Zusammensetzung umfassend die erfindungsgemäßen Stärkederivate sowie deren Verwendung zur klinischen, insbesondere parenteralen Anwendung zur Blutplasmasubstitution; Hämodilution sowie zur Verbesserung der Makround Mikrozirkulation.

### Hintergrund der Erfindung

Als künstliche kolloidale Volumenersatzmittel werden oftmals Lösungen von Gelatine oder Polysacchariden, wie beispielsweise Dextran und Hydroxyethylstärke (HES) eingesetzt. Bislang galt es, die gewünschten Wirkungen von Volumenersatzmitteln gegen die im hohen Maße auftretenden unerwünschten Nebenwirkungen der einzusetzenden Substanzen abzuwägen.

Gelatine-Lösungen werden für den Volumenersatz ausschließlich aus Rinder-Kollagen hergestellt. Neben dem, in jüngerer Vergangenheit intensiv diskutierten, potentiellen Risiko einer Infektion mit der bovinen spongioformen Encephalopathie (BSE) resultiert aus dem tierischem Rohstoff auch eine höhere Inzidenz von anaphylaktoiden/anaphylaktischen Nebenwirkungen.

Dextrane haben aufgrund ihrer hohen Rate an schweren Unverträglichkeitsreaktionen deutlich an Bedeutung verloren und wurden durch die verschiedenen Stärkelösungen nahezu vollständig verdrängt. Auch die zur Vermeidung der Unverträglichkeitsreaktionen eingeführte Vorbehandlung der Patienten mit einem monovalenten Dextran-Hapten konnte den Rückgang der Dextrananwendung zugunsten der HES-Präparate nicht mehr stoppen.

Das zunehmende Wissen über mögliche Vor- und Nachteile von Kolloiden und deren strukturelle Ursachen haben die Entwicklung neuer HES-Spezifikationen gefördert, die zunehmend den klinischen Erfordernissen angepasst werden konnten. Dennoch sind auch die verschiedenen HES-Lösungen nicht frei von Nebenwirkungen, die besonders nach hochdosierter und wiederholter Applikation zu Tage treten:

Beeinträchtigungen der Hämostase, die vorwiegend bei den älteren hochmolekularen, hochsubstituierten HES-Lösungen beobachtet wurden, konnten durch die Entwicklung der mittelmolekularen, mittel- und niedrigsubstituierten HES-Lösungen minimiert aber nicht eliminiert werden.

Die im Zusammenhang mit HES diskutierten Nierenfunktionsstörungen konnten bisher nicht eindeutig bewiesen werden.

Die Speicherung von HES in Zellen des mononukleär phagozytären Systems (MPS; früher RES) muss als derzeit größtes Problem angesehen werden. Wiederholt wurde dabei über langdauernden, therapieresistenten Pruritus (Juckreiz) berichtet, welcher, nach bisherigen Erkenntnissen, seine Ursache in der beschriebenen Persistenz von HES im MPS der Haut zu haben scheint. Inwieweit diese Speicherphänomene zugleich das Immunsystem stören ist ebenfalls noch nicht endgültig geklärt.

Das Nebenwirkungsprofil der derzeitig eingesetzten Hydroxyethylstärkelösungen wird beispielsweise von Laubenthal (Laubenthal, H. und Sirtl, C. (2001) Hydroxyethylstärke. In: Volumenersatztherapie. Hg. Boldt, J., Georg Thieme Verlag Stuttgart (2001) Seiten 80-95) ausführlich beschrieben.

Ein auf Stärkeester basierendes Volumenersatzmittel und dessen Herstellung wird in der WO-A-93/01217 offenbart. Eine daraus hervorgegangene hoffnungsvolle Entwicklung war eine Acetylstärke, die neben einer fehlenden Antigenität eine vollständige Elimination und somit keine Speicherphänomene versprach. Der klinische Einsatz solcher Acetylstärkelösungen scheiterte jedoch an der galenischen Instabilität der Präparate.

Es besteht daher noch immer ein dringender Bedarf an kolloidalen Volumenersatzmitteln, die
- eine gewünschte Wirkung auf das Kreislaufsystem erzielen;
- eine der Indikation angepassten Verweildauer im Gefäßsystem besitzen;
- eine rasche und vollständige Elimination aus dem Körper ermöglichen und
- zudem nichttoxisch, nichtantigen sowie frei von schweren Nebenwirkungen sind.

Insbesondere sollten diese Mittel keine substanzspezifische Beeinträchtigung der Blutgerinnung aufweisen und die Funktion von Organen insbesondere der Niere nicht beeinträchtigen.

Thompson et al. (Thompson, W. L., Britton, J. J., Walton, R. P. (1962), J. Pharmacol. Exptl. Therap. 136, 125-132.) erwähnten erstmals eine mögliche Verwendung von Carboxymethylstärke als Blutvolumenersatzmittel. Die Autoren beobachteten jedoch eine moderate Toxizität der getesteten Substanz in Tierversuchsreihen. Aus heutiger Sicht basierte diese Toxizität auf der hohen Molmasse und auf dem uneinheitlichen Substitutionsmuster des eingesetzten Stärkederivates. Eine chinesische Forschungsgemeinschaft (Plasma Substitute Jt. Res. Group (1978), Hua Hsueh Hsueh Pao 36, 49-76.) bewies in jüngerer Vergangenheit die Verträglichkeit von Carboxymethylstärke als Blutplasmaersatzstoff. Derartige Anwendungen sind beispielsweise in der EP-A-1078636 offenbart.

Wang (Wang, Y. (1982), Proc. Sino-Am. Symp., 55-61.) beschrieb die Herstellung und Verwendung von Carboxymethylstärken bzw. Carboxymethylamylosen mit Molmassen von 50.000 bis 70.000 Dalton als Plasmaersatzmittel. Des Weiteren existieren Verfahren zur Herstellung von carboxymethylierten Hydroxyethylstärke-Konjugaten, welche als injizierbare Kontrastmittel für die Kernspintomographie Verwendung finden (WO-A-99/42139).

Die US-A-6,479,468 beschreibt die Synthese von Polysacchariden mit verbesserten biologischen Eigenschaften, welche zu Injektionszwecken und zur Bildung von Arzneistoff-Konjugaten geeignet sind. Das Verfahren beinhaltet eine Periodat-Oxidation von Stärke mit anschließender Reduktion der entstandenen Aldehydgruppen. Durch die Periodat Oxidation erfolgt eine Spaltung der Bindung zwischen C-Atom 2 und 3 der Glucoseeinheiten.

Die meisten Veretherungen von Stärken laufen unter Basenzugabe in organischen Lösungsmitteln (größtenteils niedere Alkohole und Ketone) in einem heterogenen Reaktionssystem ab. Hierbei bleibt die granuläre Struktur der Stärke weitestgehend erhalten. Das zweiphasige System aus Stärkekörnern und Reaktionsmedium ermöglicht eine einfache Abtrennung des festen Produktes, zudem ist der granuläre Charakter des Stärkederivates für die meisten Anwendungen erwünscht.

Synthesen in organischen Lösungsmitteln wie beispielsweise in Dimethylsulfoxid, die eine homogene Reaktionsführung in Lösung ermöglichen sind nur von geringer technischer Bedeutung, weil die Produkte schwer zu isolieren sind und das Lösungsmittel aufwendig entfernt werden muss. Für eine pharmazeutische Anwendung der Stärkederivate ist das ein großer Nachteil, da hier besonders hohe Anforderungen an die Reinheit der Präparate gestellt werden.

Die Herstellung von Carboxymethylstärken in wässrigem Milieu wird in der Literatur nur unzureichend behandelt. In den vereinzelten Beispielen werden nur native Stärken unterhalb der Gelatinisierungstemperatur modifiziert, um einen Erhalt der granulären Struktur zu gewährleisten. Unter dem gleichen Vorbehalt werden in wässrigen Reaktionsmedien lediglich geringe Substitutionsgrade angestrebt, da ansonsten wegen einer zunehmenden Hydrophilierung eine unerwünschte Verkleisterung des Reaktionsansatzes unvermeidbar wäre. Die wenigen literaturbekannten Synthesen in ausschließlich wässrigen Systemen, werden z.B. von Hofreiter (Hofreiter, B. T. (1986), Chapter 11: Miscellaneous Modifications. In: Modified Starches: Properties and Uses. Ed. Wurzburg, O. B., CRC Press Florida, 179-196) beschrieben und finden überwiegend in Suspension statt und sind hinsichtlich der Substitutionsgrade der Stärkederivate eng limitiert.

Die bekannten heterogenen Synthesen von Carboxymethylderivaten aus granulären Stärken haben insgesamt den Nachteil, dass die Verteilung der Substituenten entlang der Polymerketten sehr uneinheitlich ist. Das Phänomen der uneinheitlichen, clusterartigen Substituentenverteilung bei heterogenen Synthesen von Stärkederivaten wird beispielsweise von Richardson et al. (Richardson, S., Nilsson, G. S., Bergquist, K.-E., et al. (2000), Carbohydrate Research 328, 365-373) beschrieben.

In jedem Stärkemolekül (Amylopektin oder Amylose) existiert ein terminaler Glucoserest in Form eines cyclischen Halbacetals. Für dieses Halbacetal stellt sich in Lösung ein spezifisches Geichgewicht der beiden anomeren Formen der Glucose ein. Diese Mutarotation verläuft über eine offenkettige Aldehydform. Bei gleichkonzentrierten (w/v) Stärkelösungen ist die Menge der freien reduzierenden Aldehydgruppen von der mittleren Kettenlänge der Stärkederivate abhängig. Je kurzkettiger die Stärkederivate sind, desto größer ist die Anzahl der terminalen Glucoseeinheiten und folglich steigt auch die Anzahl der Aldehydgruppen. Die relativ hohe Reaktivität dieser Aldehydgruppe führt oftmals zu unerwünschten Nebenreaktionen wie z.B. der Maillard- und/oder der Aldolreaktion sowie einer Reihe von intramolekularen Umlagerungen. Diese Nebenreaktionen sind u.a. für eine herabgesetzte Lagerfähigkeit der Blutplasmaersatzstoffe verantwortlich. Insbesondere die thermische Sterilisation der Präparate, die aufgrund der notwendigen Sterilität und Pyrogenfreiheit unumgänglich ist, forciert die Nebenreaktionen und führt oftmals zu Verfärbungen der Produkte.

Für die medizinische Anwendung kann es von Vorteil sein, zu der Lösung des Stärkederivates, neben den physiologischen Konzentrationen eines natürlichen Elektrolytenprofils (NaCl, MgCl₂, CaCl₂, KCl), bestimmte niedermolekulare Substanzen wie beispielsweise metabolisierbare Anionen (Lactat, Acetat, Malat, etc.) und/oder Aminosäuren hinzuzufügen. Diese niedermolekularen Zusätze erhöhen leider oftmals das Ausmaß der Nebenreaktionen, so dass bei der Herstellung solcher Lösungen bisher gewisse galenische Einschränkungen beachtet werden mussten.

Somit weisen ausgerechnet die zur Blutplasmasubstitution bevorzugt verwendeten kurzkettigen Stärkederivate die geringste Stabilität auf.

Da eine Vielzahl der Nebenreaktionen basenkatalysiert ablaufen, lösen die bislang im Markt erhältlichen stärkebasierten Blutplasmaersatzstoffe das Problem durch eine Verschiebung des pH-Wertes in den schwach sauren Bereich von pH 4 bis 6. Nachteilig erweist sich bei diesen Lösungen, dass der pH-Wert deutlich von dem des Blutes abweicht, welches mit einem pH von 7,4 ± 0,03 schwach basisch ist. Folglich kann die Gabe der Blutplasmaersatzstoffe leicht zu einer Übersäuerung des Blutes (Azidose) führen, die eine zusätzliche schwere Belastung des Notfallpatienten darstellt.

Die aus dem Stand der Technik resultierende und der Erfindung zugrundeliegende Aufgabe ist die Bereitstellung von physiologisch wirksamen Stärkederivaten mit guter Verträglichkeit, Lagerfähigkeit und Sterilisierbarkeit unter physiologischen Bedingungen.

Überraschenderweise wurde nun gefunden, dass eine Modifikation sämtlicher Aldehydgruppen der terminalen Glucosereste der Polysaccharidkette die zuvor beschriebenen unerwünschten Nebenreaktionen unterbindet.

Bei einer vollständigen Modifikation der terminalen Aldehydfunktionen der erfindungsgemäßen Carboxymethylstärke resultierte eine außerordentliche Stabilität gegenüber den oben beschriebenen Verhältnissen, die Nebenreaktionen begünstigen oder hervorrufen. Die Modifikation der terminalen Aldehydgruppen erfolgt dabei durch eine selektive Reduktion zum Zuckeralkohol oder durch eine selektive Oxidation zur Aldonsäure, wobei die Reduktion eine besonders bevorzugte Variante der Modifkation darstellt. Eine selektive Umsetzung mit Substanzen wie beispielsweise Natriumcyanid (Cyanhydrin-Reaktion), die ein stabiles Reaktionsprodukt mit den Aldehydgruppen bilden, ist ebenso zweckmäßig.

Anhand enzymatischer Untersuchungen und *in-vitro* Tests mit Humanblut konnte unerwarteterweise gezeigt werden, dass die biologische und physiologische Verträglichkeit der so behandelten Stärkederivate nicht beeinflusst wird.

Die erfindungsgemäße Lösung der Aufgabe ist daher ein Verfahren zur Herstellung von Stärkederivaten umfassend die Schritte
(i) der Molekulargewichtsanpassung der eingesetzten Stärke durch partielle Hydrolyse;
(ii) der Derivatisierung,
(iii) der Aufreinigung und/oder Isolierung,
dadurch gekennzeichnet, dass zwischen der Molekulargewichtsanpassung (i) und der Derivatisierung (ii) alle Aldehydgruppen vollständig modifiziert werden.

Eine weitere erfindungsgemäße Ausführungsform betrifft ein Stärkederivat, erhältlich nach diesem Verfahren.

Eine dritte Ausführungsform der Erfindung betrifft eine pharmazeutische Zusammensetzung umfassend wenigstens ein erfindungsgemäßes Stärkederivat.

Eine vierte erfindungsgemäße Ausführungsform betrifft die Verwendung des zuvor beschriebenen Stärkederivates zur Herstellung eines Arzneimittels zur Blutvolumentherapie, zur Verbesserung der Mikrozirkulation zur Hämodilution und zum Blutvolumenersatz.

Weitere erfindungsgemäße Ausführungsformen ergeben sich aus den abhängigen Ansprüchen.

### Detaillierte Beschreibung der Erfindung

Das erfindungsgemäße Herstellungsverfahren ist dadurch gekennzeichnet, dass sämtliche Verfahrensschritte vorzugsweise in wässriger Lösung oder Suspension stattfinden. Dabei liegt in der Regel spätestens nach dem Schritt der Molekulargewichtsanpassung ein homogenes System vor.

Als Ausgangsmaterialien können granuläre, native Stärken, wie z.B. Wachsmaisstärke, Maisstärke, Amylomaisstärke, Kartoffelstärke, etc., sowie partiell durch enzymatische und/oder säurehydrolytische Reaktionen abgebaute Stärken und/oder isolierte Stärkefraktionen wie Amylose oder Amylopektin dienen. Erfindungsgemäß können ebenfalls bereits derivatisierte Stärken, wie z.B. Hydroxyethylstärke eingesetzt und erneut modifiziert werden. Diese möglichen Ausgangsmaterialien unterschiedlicher Zusammensetzung und Provenienz werden im Folgenden als "Stärke" bezeichnet.

Im ersten Schritt (i) des erfindungsgemäßen Verfahrens erfolgt eine Molekulargewichtsanpassung der eingesetzten Stärke. Vorzugsweise erfolgt die Anpassung des Molekulargewichtes durch eine säurekatalysierte Spaltung der glykosidischen Bindungen unter Anwendung von Mineralsäuren. Alternativ kann ebenso durch den Einsatz von stärkeabbauenden Enzymen, wie beispielsweise α-Amylasen (EC 3.2.1.1) ein gewünschtes Molekulargewicht erreicht werden.

Alternativ oder unterstützend kann die Molekulargewichtsanpassung durch Ultraschallabbau, Mikrowellenbehandlung, Einwirkung von Scherkräften oder durch die Kombination der verschiedenen Methoden erfolgen.

Bei der partiellen Säurehydrolyse wird die Stärke in Wasser in einer Konzentration von bis zu 40 Gew.-%, vorzugsweise 5 bis 30 Gew.-%, besonders bevorzugt 10 bis 20 Gew.-% gelöst bzw. suspendiert. Die Reaktionsmischung wird unter kräftigem Rühren auf eine Temperatur von 50 bis 95 °C, vorzugsweise 60 bis 90 °C, besonders bevorzugt 70 bis 90 °C erwärmt.

Bei einem Einsatz von granulären Stärken wird eine Temperatur oberhalb des für jede Stärkesorte individuellen Verkleisterungsbereichs eingestellt, um ein Aufbrechen der Stärkekörner zu gewährleisten. Nach Erreichen der maximalen Homogenität wird die Hydrolyse durch die Zugabe einer entsprechenden Menge Mineralsäure unter ständigem Rühren gestartet. Geeignete Mineralsäuren sind ausgewählt aus der Gruppe bestehend aus Salzsäure, Phosphorsäure, Salpetersäure, Perchlorsäure und Schwefelsäure, vorzugsweise wird Salzsäure verwendet. Eine Verwendung starker organischer Säuren wie beispielsweise Trifluor- oder Trichloressigsäure ist prinzipiell ebenfalls möglich. Das Fortschreiten der Hydrolyse lässt sich durch Viskositätsmessungen und/oder durch begleitende mehrfache Bestimmung der Molekulargewichtsverteilung mittels GPC-MALLS verfolgen.

Ist das gewünschte Molekulargewicht erreicht, welches erfindungsgemäß im Bereich von 20.000 bis 1.000.000, vorzugsweise 40.000 bis 500.000, besonders bevorzugt 60.000 bis 200.000 liegt, so wird die Hydrolyse durch Neutralisation mit einer Base, vorzugsweise Natronlauge beendet.

In Abhängigkeit von der Natur der eingesetzten Stärke kann es neben der Molekulargewichtsanpassung erforderlich sein, den Verzweigungsgrad zu modifizieren. Der Verzweigungsgrad der erfindungsgemäßen Stärken liegt im Bereich von 0 bis 6 %, vorzugsweise 2 bis 5,5 %, besonders bevorzugt 3 bis 5 %. Die Entzweigung der Stärken kann während oder im Anschluss an die Molekulargewichtsanpassung (i) erfolgen. In der Regel wird nach der Beendigung der Hydrolyse die Stärkesuspension/-lösung mit Pullulanase (EC 3.2.1.41) und/oder Isoamylase (EC 3.2.1.68) behandelt und die Stärke in einem gewünschten Ausmaß entzweigt. Erfindungsgemäß werden die Enzyme in einer Menge von 100 bis 5000 U/kg Stärke eingesetzt, bevorzugt sind Mengen von 500 bis 4000 U/kg, besonders bevorzugt von 1000 bis 1500 U/kg. Die Aktivität der Enzyme ist dabei so definiert, dass eine Einheit [1U] der Enzymmenge entspricht, die 1 µmol Substrat pro Minute unter festgelegten Bedingungen umsetzt.

Die Entzweigung findet unter den für das Enzymsystem optimierten Bedingungen statt (pH, Temperatur, Dauer der Inkubation). Solche Reaktionsbedingungen sind beispielsweise in der EP-A-0372184 im Zusammenhang mit Pullulanase als Enzym offenbart.

Nach der Anpassung des Molekulargewichtes und des Verzweigungsgrades wird das Reaktionssystem auf einen pH-Wert von 4 bis 12, vorzugsweise 6 bis 10, besonders bevorzugt 8 bis 9 eingestellt.

In einer bevorzugten Ausführungsform der Erfindung erfolgt nun im gleichen Reaktionsansatz eine Modifikation sämtlicher terminaler Aldehydfunktionen, um weitere ungewollte Nebenreaktionen zu unterbinden und das am Ende resultierende Stärkederivat für die klinische Anwendung als Plasmaersatz in einer physiologischen Lösung zu stabilisieren. Die physiologische Verträglichkeit der Stärken wird durch diesen Eingriff überraschenderweise nicht beeinträchtigt.

Eine Modifikation der terminalen Aldehydfunktionen kann durch eine selektive Oxidation mit geeigneten Oxidationsmitteln erfolgen. Hierbei wird beispielsweise durch Zutropfen einer Iod-Lösung in die schwach alkalische Stärke-Lösung (unter Bildung von hypoiodiger Säure) eine Oxidation der Aldehydfunktion zur Säurefunktion erreicht. Geeignete Oxidationsmittel können somit ausgewählt sein aus der Gruppe bestehend aus hypochloriger Säure, hypobromiger Säure, hypoiodiger Säure sowie deren Salzen (Hypohalogenite), Polysulfiden und Anthrachinonen. In einer Alternativen Ausführungsform können die Oxidationsmittel an wasserunlöslichen inerten Polymeren festphasen gebunden vorliegen und somit von den Reaktionsprodukten leicht abtrennbar und regenerierbar sind.

Die Vollständigkeit der Oxidation kann durch den kalorimetrischen Nachweis mit 3,5-Dinitrosalicylsäure erfolgen (vgl. Grassl, M., Walter, H. E. (1983), a-Amylase. In: Methods of Enzymatic Analysis. Ed. Bergmayer, H. U., 3rd Ed. Vol. 2A, Verlag VCH Weinheim, 151-152.). Vollständig bedeutet in diesem Zusammenhang, dass im Vergleich zur Blindprobe mit der zuvor erwähnten Methode keine reduzierende Wirkung mehr nachgewiesen werden kann.

Ebenfalls möglich ist eine weitere selektive Umsetzung der Aldehydfunktion mit geeigneten Reagenzien wie beispielsweise Natriumcyanid. Hierdurch werden die störenden Aldehydfunktionen durch Cyanhydrin-Bildung eliminiert und die neu eingeführte Nitrilfunktion im weiteren Verlauf der Stärkefunktionalisierung zur Säurefunktion verseift. Die Vollständigkeit der Nitrilverseifung wird durch NMR- oder IR-Spektroskopie überprüft. Vollständig bedeutet in diesem Zusammenhang, dass keine Nitrilfunktion mit den zuvor erwähnten Methoden mehr detektierbar ist.

In einer weiteren erfindungsgemäßen Ausführungsform kann eine Reaktion der Aldehydfunktion mit Aminen, Aminosäuren, Peptiden und Proteinen erfolgen. Die Reaktion wird durch geeignete Kopplungsreagenzien wie z.B. Carbodiimiden begünstigt.

Eine weitere bevorzugte Möglichkeit der Modifikation ist die vollständige Reduktion der terminalen Aldehydfunktionen zur korrespondierenden Alkoholfunktion mit geeigneten Reduktionsmitteln. Als besonders bevorzugte Reduktionsmittel sind hierbei komplexe Metallhydride aus der Gruppe bestehend aus Natriumborhydrid, Kaliumborhydrid, Lithiumborhydrid sowie Natriumcyanoborhydrid zu nennen. Deuterierte und Tritierte Varianten dieser Verbindungen sind ebenfalls geeignet. Bei der Durchführung der Reduktion wird das Reduktionsmittel in einem Überschuss von 10 bis 1000 Äq., bevorzugt von 20 bis 200 Äq., besonders bevorzugt von 30 bis 60 Äq. bezogen auf die im Reaktionsansatz vorhande molare Menge an terminalen Aldehydfunktionen eingesetzt. Die Zugabe des Reduktionsmittels findet bei einer Temperatur von 10 bis 90 °C, bevorzugt von 20 bis 80 °C, besonders bevorzugt von 60 bis 70 °C statt. Im Anschluss wird die Reaktionsmischung so lange gerührt, bis alle terminalen Aldehydfunktionen reduziert worden sind. Die Überprüfung der Vollständigkeit der Reduktion kann durch den kolorimetrischen Nachweis mit 3,5-Dinitrosalicylsäure erfolgen (Grassl, M., Walter, H. E. (1983), a-Amylase. In: Methods of Enzymatic Analysis. Ed. Bergmayer, H. U., 3rd Ed. Vol. 2A, Verlag VCH Weinheim, 151-152).

Vollständig bedeutet in diesem Zusammenhang, dass im Vergleich zur Blindprobe mit der zuvor erwähnten Methode keine reduzierende Wirkung mehr nachgewiesen werden kann.

Die nachfolgende Derivatisierung (ii) der Stärken gemäß der vorliegenden Erfindung erfolgt an den freien Hydroxyl-Gruppen unter Bildung von Carboxyalkylstärken, Stärkeestern oder Hydroxyalkylstärken.

Zur Herstellung der Carboxyalkylstärken wird die Reaktionsmischung durch Zugabe von Basen, vorzugsweise von Natriumhydroxid stark alkalisch eingestellt (pH 10 bis 14) und anschließend mit bis zu 3,0 Äq., bevorzugt 0,2 bis 2,0 Äq., besonders bevorzugt 0,3 bis 1,0 Äq., bezogen auf die molare Menge der Monomereinheiten des Polysaccharids, des Carboxyalkylierungsreagenzes versetzt. Erfindungsgemäß eignen sich Carboxyalkylierungsreagenzien, die ausgewählt sind aus der Gruppe bestehend aus Monochloressigsäure, Monobromessigsäure, Monoiodessigsäure oder deren Salzen. Besonderer Vorzug gilt der Monochloressigsäure und dessen Natriumsalz. Der Einsatz anderer α-Halogencarbonsäuren bzw. α-Halogendicarbonsäuren (wie z.B. Chlormalonsäuredimethylester) ist ebenfalls möglich. Je nach Art der zugrundeliegenden Stärke und den jeweiligen stöchiometrischen Verhältnissen der Reaktionspartner lassen sich bei einer Temperatur im Bereich von 10 bis 90 °C, vorzugsweise 20 bis 80 °C, besonders bevorzugt 30 bis 70 °C und einer Reaktionszeit von 2 bis 24 Stunden, vorzugsweise 4 bis 14 Stunden, besonders bevorzugt 6 bis 8 Stunden, unterschiedliche Substitutionsgrade und -muster erzielen. Die zur Durchführung von Veretherungen zu treffenden Maßnahmen sind dem Fachmann aus dem Stand der Technik, wie beispielsweise von Engelskirchen (Engelskirchen, K. (1987), Umwandlung von Stärke. In: Houben-Weyl. Methoden der organischen Chemie, E20/3, Georg Thieme Verlag Stuttgart, 2135-2175) beschrieben, bekannt.

Alternativ können die partiell hydrolysierten und terminal modifizierten Stärken auch zu Stärkeestern umgesetzt werden. Die Veresterung erfolgt vorzugsweise unter milder Basenkatalyse bei einem konstant zu haltenden pH-Wert im Bereich von 6 bis 11, vorzugsweise 7 bis 10, besonders bevorzugt 8 bis 9 mit Mono-, Di- oder Tricarbonsäuren sowie deren Halogenide oder Anhydride.

Geeignete Säureanhydride leiten sich von aliphatischen Monocarbonsäuren mit 2 bis 8, vorzugsweise 2 bis 6, besonders bevorzugt 2 bis 4 Kohlenstoffatomen oder von aliphatischen Dicarbonsäuren mit 2 bis 8, vorzugsweise 2 bis 6, besonders bevorzugt 2 bis 4 Kohlenstoffatomen ab. Die Monocarbonsäuren sind vorzugsweise ausgewählt aus der Gruppe bestehend aus Essigsäure, Propionsäure, Buttersäure, Isobuttersäure, wobei Essigsäure besonders bevorzugt verwendet wird. Die Dicarbonsäuren sind vorzugsweise ausgewählt aus der Gruppe bestehend aus Oxalsäure, Malonsäure, Bernsteinsäure, Glutarsäure und Adipinsäure, wobei Bernsteinsäure und Glutarsäure besonders bevorzugt verwendet werden.

Des Weiteren sind Mono-, Di- und Tricarbonsäuren zu berücksichtigen, die zusätzlich eine oder mehrere Hydroxylfunktionen aufweisen. Insbesondere sind hierunter Substanzen wie Glykolsäure, Milchsäure, Äpfelsäure oder Zitronensäure zu verstehen.

Geeignete Carbonsäurehalogenide sind ausgewählt aus der Gruppe der Chloride, Bromide oder Iodide. Bevorzugt sind die Chloride der zuvor genannten Mono- und Dicarbonsäuren. Die zur Durchführung von Veresterungen zu treffenden Maßnahmen, sind dem Fachmann aus dem Stand der Technik, wie beispielsweise von Engelskirchen (Engelskirchen, K. (1987), Umwandlung von Stärke. In: Houben-Weyl. Methoden der organischen Chemie, E20/3, Georg Thieme Verlag Stuttgart, 2135-2175) oder in der WO-A-93/01217 beschrieben, bekannt.

Eine Veresterung kann auch direkt mit den freien Carbonsäuren erfolgen, wobei die zuvor genannten Carbonsäuren besonders geeignet sind. Hierbei werden zur Knüpfung der Esterbindung bevorzugt Kondensationsmittel aus der Klasse der Carbodiimide verwendet. Als mögliches Carbodiimid wird besonders bevorzugt eine wasserlösliche Variante wie beispielsweise N-Ethyl-N'-(3-dimethylaminopropyl)-carbodiimid Hydrochlorid (EDC) eingesetzt. Das allgemeine Prinzip der Veresterung mit Carbodiimiden wird in Standardlehrbüchern der Organischen Chemie beschrieben (Christen, H. R., Vögtle, F. (1992), Organische Chemie: Von den Grundlagen zur Forschung, Band 1, 2. Aufl., Otto Salle Verlag Frankfurt am Main.).

Die Darstellung von Hydroxyalkylstärken erfolgt im allgemeinen durch Umsetzung der Stärken mit Epoxiden, besonders bevorzugt sind hierbei Ethylenoxid oder Propylenoxid, daneben ist die Verwendung von 2-Chlorethanol ebenso bevorzugt. Die zur Durchführung von Hydroxyalkylierungen zu treffenden Maßnahmen sind dem Fachmann aus dem Stand der Technik, wie beispielsweise von Engelskirchen (Engelskirchen, K. (1987), Umwandlung von Stärke. In: Houben-Weyl. Methoden der organischen Chemie, E20/3, Georg Thieme Verlag Stuttgart, 2135-2175) oder in der EP-A-0402724 beschrieben, bekannt.

Bei den erfindungsgemäßen Stärkederivaten handelt es sich bevorzugt um Hydroxyethylstärke (HES), Carboxymethylstärke (CMS), Carboxymethyl-hydroxyethylstärke (CM-HES) und Succinylstärke (SUS) wobei polyanionische Stärkederivate, wie beispielsweise Carboxymethylstärke (CMS) besonders bevorzugt sind.

Um die erforderliche physiologische Verträglichkeit und die gewünschte relativ kurze Verweilzeit der Stärkederivate im Blutkreislauf des Patienten zu erzielen, ist ein möglichst gleichmäßiges Substitutionsmuster der Stärkederivate notwendig. Ein einheitliches Substitutionsmuster wird von mehreren Parametern beschrieben und ist dann gegeben wenn folgende Voraussetzungen erfüllt sind:
a) Der molare Substitutionsgrad (MS) und der Substitutionsgrad (DS) ähnlich, vorzugsweise identisch ist. Bei identischen Werten können Mehrfachsubstitutionen von Monomereinheiten ausgeschlossen werden.
   Der Substitutionsgrad MS ist definiert als die durchschnittliche Anzahl von Substituenten pro Anhydroglucoseeinheit. Hingegen ist der Substitutionsgrad DS definiert als das Verhältnis der substituierten Anhydroglucoseeinheiten gegenüber der Gesamtzahl aller Anhydroglucoseeinheiten.
b) Die Verteilung der Substituenten auf die unterschiedlichen Hydroxylgruppen der Anhydroglucoseeinheiten (an C-Atom 2, 3 und 6) möglichst einheitlich ist. Da Substitutionen an den theoretisch möglichen Positionen der Monomere einen unterschiedlichen Einfluss auf die Pharmakokinetik des Produktes haben, ist eine hohe Regioselektivität erstrebenswert.
c) Die Verteilung der Substituenten entlang des polymeren Grundgerüstes möglichst gleichförmig ist und nicht wie im Stand der Technik vorzufinden uneinheitlich und clusterartig ist.

Eine gleichmäßige Verteilung entlang der Stärkeketten führt zu einer optimalen Steuerung der Pharmakokinetik und verhindert das Auftreten von Metaboliten mit hohen Substituentendichten, welche Speicherphänomene und darauf beruhende Nebenwirkungen hervorrufen würden.

Erfindungsgemäß liegen die Werte für den Substitutionsgrad MS im Bereich von 0,01 bis 1,00, vorzugsweise von 0,05 bis 0,60, besonders bevorzugt von 0,1 bis 0,45 und für den Substitutionsgrad DS im Bereich von 0,01 bis 1,00, vorzugsweise von 0,05 bis 0,60, besonders bevorzugt von 0,1 bis 0,45.

Das Substitutionsmuster weist eine überwiegende Lokalisierung (> 70 %, vorzugsweise > 80 %, besonders bevorzugt > 85 %) der eingeführten Substituenten an den sekundären Hydroxylgruppen, bevorzugt an der Hydroxylgruppe an C-Atom 2, der Anhydroglucoseeinheiten auf.

Die Verteilung der Substituenten entlang den Stärkeketten ist möglichst einheitlich. Eine derartige Verteilung lässt sich nicht numerisch angeben und kann derzeit nur durch enzymatischen Abbau und anschließender Analyse der Spaltprodukte ermittelt werden. Die Substituenten liegen erfindungsgemäß jedoch mit dem größtmöglichen Abstand zueinander innerhalb des Polymers verteilt vor. Etwas vereinfacht ausgedrückt wird bei einem gegebenen Substitutionsgrad eine homogen substituierte Stärke weniger durch α-Amylasen abgebaut als dies bei heterogen substituierten Stärken der Fall wäre. Durch die einheitlichere Substitution werden persistente Fraktionen vermieden, welche beim bisherigen Stand der Technik (Hydroxyethylstärke) Nebenwirkungen wie Juckreiz verursachen. Die statistische Verteilung der Substituenten wird bei den erfindungsgemäßen Stärken durch einen möglichst geringen Substitutionsgrad und das homogene überwiegend in wässriger Lösung ablaufende Herstellungsverfahren erreicht.

Bei dem erfindungsgemäßen Herstellungsprozess handelt es sich um ein sogenanntes Eintopfverfahren bei dem sämtliche Syntheseschritte ohne die Isolierung von Zwischenprodukten vorzugsweise in wässriger Lösung ablaufen. Mit Ausnahme der Enzymreaktionen können alle Reaktionen zwischen 10 und 90 °C, bevorzugt zwischen 50 und 80 °C, besonders bevorzugt zwischen 60 und 70 °C durchgeführt werden.

Die Isolierung (iii) des vorzugsweise polyanionischen Produktes erfolgt bevorzugt über Dialyse- und/oder Ultrafiltrationstechniken und anschließender Sprüh- oder Gefriertrocknung. Man erhält so eine den klinischen Anforderungen entsprechend aufgereinigte und modifizierte Stärke.

Die Molekulargewichtsverteilung des Produktes kann über Größenausschlusschromatographie mit anschließender Streulichtmessung (GPC-MALLS) ermittelt werden. Der Substitutionsgrad, das Substitutionsmuster sowie der Verzweigungsgrad und das Maß der terminalen Aldehyd-Funktionen bzw. dessen Modifizierungsgrad wird über ¹H-NMR- oder ¹³C-NMR-Methoden bestimmt (siehe hierzu beispielsweise: Nilsson, G. S., Bergquist, K.-E., Nilsson, U., Gorton, L. (1996), Starch/Stärke 48, 352-357 sowie Bach Tuyet, L. T., Ilyama, K., Nakano, J. (1985), Mokuzai Gakkaishi 31, 8-13).

Eine Aussage über die Homogenität der Verteilung der Substituenten entlang der Polysaccharidkette kann über enzymatische Methoden getroffen werden (siehe hierzu beispielsweise: Zhang, Jingwu, Wu, Dahua (1991), Tianjin Daxue Xuebao 4, 82-87 und Richardson, S., Nilsson, G. S., Bergquist, K.-E., et al. (2000), Carbohydrate Research 328, 365-373).

Die erfindungsgemäßen Stärkederivate können in pharmazeutische Zusammensetzungen eingearbeitet werden. Zur Herstellung der pharmazeutischen Zusammensetzungen werden die erfindungsgemäßen Stärkederivate in einem physiologisch verträglichen Lösungsmittel, vorzugsweise in Wasser gelöst. Die erfindungsgemäßen Zusammensetzungen weisen einen physiologischen pH-Wert von 6 bis 8, vorzugsweise 6,5 bis 7,5, besonders bevorzugt 7,4 ± 0,3 auf. Gegenüber Zusammensetzungen des Standes der Technik zeichnen sich die erfindungsgemäßen pharmazeutischen Zusammensetzungen durch ihre hervorragende Lagerstabilität und thermische Stabilität aus, infolge derer die Lösungen problemlos und ohne Verfärbungen sterilisiert werden können.

Die erfindungsgemäße pharmazeutische Zusammensetzung enthält wenigstens 1 bis 10, vorzugsweise 3 bis 7, besonders bevorzugt 4 bis 6 Gew.-% bezogen auf die Gesamtzusammensetzung, der erfindungsgemäßen Stärkederivate.

Die Zusammensetzungen können zudem weitere Bestandteile aufweisen, die ausgewählt sind aus der Gruppe bestehend aus Glucose, Xylit, Sorbit (oder andere Mono- und Disaccharide) und aus der Gruppe der Aminosäuren vorwiegend der proteinogenen Aminosäuren. Des Weiteren können in der Zusammensatzung Kationen enthalten sein die ausgewählt sind aus der Gruppe bestehend aus Natrium (Na⁺), Kalium (K⁺), Calcium (Ca²⁺), Magnesium (Mg²⁺), Zink (Zn²⁺) oder Kombinationen der genannten Kationen sowie Anionen die ausgewählt sind aus der Gruppe bestehend aus Acetat, Lactat, Malat, Albuminat, Chlorid (Cl⁻), Carbonat (CO₃²⁻), Hydrogencarbonat (HCO₃⁻), Phosphat (PO₄³⁻), Hydrogenphosphat (HPO₄²⁻), Dihydrogenphosphat (H₂PO₄⁻) oder Kombinationen der genannten Anionen.

Die erfindungsgemäßen pharmazeutischen Zusammensetzungen weisen eine hervorragende Eignung als Plasmaersatzmittel und zur Hämodilution auf. Bei der Anwendung konnte insbesondere gegenüber den pharmazeutischen Zusammensetzungen des Standes der Technik eine Verbesserung der Fließeigenschaften des Blutes, d.h. der Durchblutung von kleineren Gefäßen und eine nur noch auf den Verdünnungseffekt zurückzuführende Beeinflussung der Blutgerinnung (d.h. keine spezifische Interferenz mit Gerinnungsparametern) beobachtet werden.

Aufgrund des beschriebenen homogenen Herstellungsverfahren sind die erfindungsgemäßen Stärkederivate gegenüber dem Stand der Technik durch eine besonders einheitliche Substitution gekennzeichnet, welche eine optimale Steuerung der Volumenwirksamkeit sowie eine Minimierung von Speicherphänomenen ermöglicht.

Die Erfindung betrifft daher neben den erwähnten pharmazeutischen Zusammensetzungen die das erfindungsgemäße Stärkederivat umfassen auch ein Verfahren zur Blutplasmasubstitution unter Verwendung der erfindungsgemäßen pharmazeutischen Zusammensetzung bzw. die Verwendung des erfindungsgemäßen Stärkederivats zur Volumentherapie, zur Verbesserung der Mikrozirkulation, zur Hämodilution oder zum Blutvolumenersatz bzw. zur Herstellung eines Arzneimittels zu diesen Zwecken.

Die Erfindung wird anhand der folgenden Beispiele weiter erläutert, ohne sie jedoch auf diese zu beschränken.

### Beispiele

### Darstellung einer Carboxymethylstärke (Mw = 190.000, MS = 0,4):

Für die erfindungsgemäße Umwandlung in einem Eintopfverfahren werden 100 g (617 mmol Anhydroglucose) Wachsmaisstärke in ein Reaktionsgefäß vorgelegt und unter Rühren mit 960 ml bidestilliertem Wasser versetzt. Die Rührfrequenz wird erhöht, um eine möglichst homogene Mischung zu erhalten. Die Temperatur des Gemisches wird dabei auf 65 °C gebracht. Die Säurehydrolyse wird durch die Zugabe von 40 ml 5 N Salzsäure gestartet. Die Reaktionsmischung verliert während der Hydrolyse zunehmend an Viskosität und wird allmählich klarer. Nach einem Zeitraum von 7 Stunden wird die Hydrolyse durch Neutralisation mit 40 ml 5 N Natronlauge beendet. Es wird eine Probe entnommen, um die kinematische Viskosität bzw. später das Molekulargewicht zu bestimmen. Es resultiert eine opaleszierende, kolloidale Lösung einer partiell hydrolysierten Stärke.

Für die folgende Reduktion der terminalen Aldehydfunktionen der Stärkemoleküle wird ein pH-Wert von etwa 9 eingestellt. Man gibt unter stetigem Rühren 0,60 g (15,9 mmol) Natriumborhydrid langsam zur Reaktionslösung. Die Lösung wird bei 65 °C für 14 Stunden gerührt, nach dieser Zeit ist keine Gasentwicklung mehr registrierbar. Man entnimmt eine Probe, um die Vollständigkeit der Reduktion wie unten beschrieben zu überprüfen. Wenn die Reduktion vollständig ist, kann die Lösung weiter umgesetzt werden, ansonsten durch eine erneute Zugabe von Natriumborhydrid nachreduziert werden.

Für die Carboxymethylierung der partiell abgebauten sowie reduzierten Stärke wird unter Rühren bei 65 °C zum Reaktionsansatz 14,8 g (370 mmol) festes Natriumhydroxid gegeben. Hierbei klärt sich der Ansatz auf und bei ausreichender Reduktion kommt es zu keiner Verfärbung mehr. Es wird für eine halbe Stunde gerührt und anschließend spatelweise 43,1 g (370 mmol) des Natriumsalzes der Monochloressigsäure in die alkalische Reaktionslösung gegeben. Man rührt den Ansatz bei 65 °C für 5 Stunden.

Schließlich wird die klare Lösung über eine D3-Glasfritte abfiltriert und nach dem Abkühlen auf einen pH-Wert von 7,4 gebracht. Die endgültige Aufarbeitung erfolgt durch Dialyse der Reaktionslösung gegen bidestilliertes Wasser (Cellulose-Dialysierschlauch, Ausschlussgrenze 20.000 Da) und anschließende Lyophilisation.

Charakteristische Daten der Carboxymethylstärke:
Ausbeute: 79,2 g farbloser Feststoff (dialysiert und lyophilisiert)
Molekulargewicht (Mw): 194000
Verzweigungsgrad: 5,4 %
Modifikationsgrad der terminalen Aldehydfunktionen: 100 %
MS: 0,42
MS(C₂+C₃): 0,37
MS(C₆): 0,05
Verhältnis MS(C₂+C₃) / MS(C₆): 7,4
Reaktionseffizienz: 70,0 %
¹H-NMR(D₂O, pD=1, TSP ext. Std.): δ = 5.83-5.52 (m, Maximum bei 5.67 ppm, H-1), 5.52-5.21 (m, Maxima bei 5.49 und 5.38 ppm, H-1), 5.31 (Schulter, H-1), 4.96 (m), 4.80 (s, HDO), 4.51-4.30 (m, Maxima bei 4.41, -CH₂COOH), 4.30-4.16 (m, Maxima bei 4.21, -CH₂COOH), 4.16-3.34 (nicht aufgel., Maxima bei 4.08, 3.85, 3.70, 3.60, 3.52 ppm), 3.40 (m)
¹³ C-NMR(D₂O, TSP ext. Std.): δ = 176.3, 102.1, 99.8, 82.1, 79.8, 77.9, 75.9, 75.2, 74.0, 73.9, 70.7, 70.2, 63.1
¹H-NMR(D₂O/D₂SO₄, TSP ext. Std.): δ = 5.37 (d, 1H, H-1(α-s), J=3.6), 5.18 (d, 1H, H-1(α-u), J=3.8), 4.82 (HDO), 4.69 (d, 1H, H-1( β-s), J=7.9), 4.61 (d, 1H, H-1(β-u), J=7.9), 4.43 (m, 2H, a,β-3-O-CH₂COOH), 4.41 (d, 1H, β-2-O-CHₐH_{b}COOH, J=17.0), 4.38 (d, 1H, β-2-O-CₐH_{b}COOH, J=17.0), 4.32 (d, 1H, α-2-O-CHₐH_{b}COOH, J=17.0), 4.29 (d, 1H, α-2-O-CHₐH_{b}COOH, J=17.0), 4.18 (m, 2H, a,β-6-O-CH₂COOH), 3.83 (dd, 1H, H-6'(β), J=2.3, J=12.5), 3.78, 3.77 (je m, H-5(α), H-6'(α), (u) und (s)), 3.71, 3.68, 3.67 (je m, H-6(α), H-6(β), H-3(α), (u) und (s)), 3.58 (dd, 1H, H-3(β-s), J=9.2, J=9.2), 3.53 (dd, 1H, H-2(α), J=3.8, J=9.9), 3.48 (dd, 1H, H-3(β-u), J=9.0, J=9.0), 3.43 (ddd, 1H, H-5(β), J=2.2, J=5.4, J=9.8), 3.38 (dd, 1H, H-4(β), J=9.7, J=9.8), 3.38 (dd, 1H, H-4(α), J=9.3, J=9.6), 3.21 (dd, 1H, H-2(β-u), J=7.9, J=9.5), 3.14 (dd, 1H, H-2(β-s), J=7.9, J=9.5).
FTIR(ATR): 3330 s, 2928 s, 1728 s, 1590 s, 1411 s, 1362 s, 1149 s, 1078 s, 1014 s

### Analytische Verfahren

### - Viskositätsmessung:

Die Viskositätsmessung erfolgt mit einem handelsüblichen Ubbelohde-Kapillar-Viskosimeter. Die gemessenen kinematischen Viskositäten korrelieren mit dem mittleren Molekulargewicht (Mw) des Stärkehydrolysats.

### - Molekulargewichtsbestimmung:

Die Bestimmung der Molekulargewichte erfolgt über Streulichtmessungen (MALLS) mit vorgeschalteter Größenausschlusschromatographie (GPC).

### - Bestimmung von MS und der partiellen Substitutionsgrade MS(C2+C3) und MS(C6):

Der molare Substitutionsgrad (MS) und die Substitutionsgrade an der Hydroxylgruppe an C-Atom 6 (MS(C6)) und an C-Atom 2 und 3 (MS(C2+C3) lassen sich nach saurer Totalhydrolyse des Stärkeethers über ¹H-NMR-Spektroskopie bestimmen. Das Verfahren wird beispielsweise von Bach Tuyet (Bach Tuyet, L. T., Ilyama, K., Nakano, J. (1985), Mokuzai Gakkaishi 31, 8-13.) beschrieben.

### - NMR-spektroskopische Bestimmung des Verzweigungsgrades:

Der Grad der α-1,6-glykosidischen Verzweigungen kann gemäß Nilsson et al. (Nilsson, G. S., Bergquist, K.-E., Nilsson, U., Gorton, L. (1996), Starch/Stärke 48, 352-357.) über ¹H-NMR-Spektroskopie bestimmt werden.

### - NMR-spektroskopische Bestimmung der terminalen Aldehydfunktionen:

Die Vollständigkeit der Modifizierung der terminalen Aldehydfunktionen kann durch das Fehlen des spezifischen Aldehydsignals von terminalen Glucoseresten überprüft werden. Hierbei wird das von Nilsson et al. (Nilsson, G. S., Bergquist, K.-E., Nilsson, U., Gorton, L. (1996), Starch/Stärke 48, 352-357.) beschriebene ¹H-NMR-spektroskopische Verfahren angewendet.

### - Kolorimetrische Bestimung der terminalen Aldehydfunktionen:

Die Vollständigkeit der Modifizierung der terminalen Aldehydfunktionen kann durch das Fehlen der reduzierenden Wirkung von terminalen Glucoseresten überprüft werden. Hierbei wird ein auf der Farbreaktion mit 3,5-Dinitrosalicylsäure basierendes photometrisches Verfahren angewendet (Grassl, M., Walter, H. E. (1983), α-Amylase. In: Methods of enzymatic analysis. Ed. Bergmayer, H. U., 3rd Ed. Vol. 2A, Verlag VCH Weinheim, 151-152).

## Patentansprüche

**1.** Verfahren zur Herstellung von Stärkederivaten, umfassend die Schritte
(i) der Molekulargewichtsanpassung der eingesetzten Stärke durch partielle Hydrolyse;
(ii) der Derivatisierung; und
(iii) der Aufreinigung und/oder Isolierung,
**dadurch gekennzeichnet, dass** zwischen dem Schritt der Molekulargewichtsanpassung (i) und der Derivatisierung (ii) alle Aldehydgruppen vollständig modifiziert werden.

**2.** Verfahren nach Anspruch 1, **dadurch gekennzeichnet, dass** die Verfahrensschritte (i) bis (iii) und die Modifizierung der Aldehydgruppen in wässriger Lösung oder Suspension erfolgen.

**3.** Verfahren nach irgendeinem der Ansprüche 1 bis 2, **dadurch gekennzeichnet, dass** die Verfahrensschritte (i) bis (iii) und die Modifizierung der Aldehydgruppen ohne Isolierung von Zwischenprodukten durchgeführt werden.

**4.** Verfahren nach irgendeinem der Ansprüche 1 bis 3, **dadurch gekennzeichnet, dass** die Molekulargewichtsanpassung (i) der Kohlenhydrate durch säurekatalysierte oder enzymatische Hydrolyse, Ultraschallabbau, Mikrowellenbehandlung, Scherkräfte oder durch die Kombination der verschiedenen Methoden erfolgt.

**5.** Verfahren nach irgendeinem der Ansprüche 1 bis 4, **dadurch gekennzeichnet, dass** die eingesetzten Stärken ausgewählt sind aus der Gruppe bestehend aus Maisstärke, Wachsmaisstärke, Amylomaisstärke, Kartoffelstärke, Weizenstärke, Reisstärke, Milostärke, Tapiokastärke, amylopektinreiche Kartoffelstärke, Sorghumstärke, Erbsenstärke, Süßkartoffelstärke, Bananenstärke, Sagostärke, Stärkefraktionen (Amylose oder Amylopektin), dünnkochende Stärken (enzymatisch oder säurehydrolytisch vorbehandelt), dextrinierte Stärken, Stärken aus transgenen Pflanzen, Stärkeether, Hydroxyethylstärke, Hydroxypropylstärke, Carboxymethylstärke.

**6.** Verfahren nach irgendeinem der Ansprüche 1 bis 5, umfassend einen enzymatischen Entzweigungsschritt.

**7.** Verfahren nach Anspruch 6, **dadurch gekennzeichnet, dass** das zur Entzweigung eingesetzte Enzym Pullulanase (EC 3.2.1.41) oder Isoamylase (EC 3.2.1.68) bzw. eine Kombination aus beiden ist.

**8.** Verfahren nach irgendeinem der Ansprüche 1 bis 7, **dadurch gekennzeichnet, dass** die Modifizierung der terminalen Aldehydgruppen durch Reduktion zur korrespondierenden Alkoholfunktion erfolgt.

**9.** Verfahren gemäß Anspruch 8, wobei als Reduktionsmittel vorzugsweise Schwefelwasserstoff, Lithiumaluminiumhydrid, komplexe Hydride und/oder Metallhydride, die ausgewählt sind aus der aus der Gruppe bestehend aus Lithiumborhydrid, Natriumborhydrid, Kaliumborhydrid, Natriumcyanoborhydrid und sulfidiertes Natriumborhydrid verwendet werden.

**10.** Verfahren nach irgendeinem der Ansprüche 8 bis 9, **dadurch gekennzeichnet, dass** die Reduktionsmittel an wasserunlöslichen inerten Polymeren festphasengebunden vorliegen und somit von den Reaktionsprodukten leicht abtrennbar und regenerierbar sind.

**11.** Verfahren nach irgendeinem der Ansprüche 1 bis 7, **dadurch gekennzeichnet, dass** die Modifizierung der terminalen Aldehydgruppen durch Oxidation zur korrespondierenden Säurefunktion erfolgt.

**12.** Verfahren gemäß Anspruch 11, wobei das Oxidationsmittel ausgewählt ist aus der Gruppe bestehend aus hypochloriger Säure, hypobromiger Säure, hypoiodiger Säure sowie deren Salzen (Hypohalogenite), Polysulfiden und Anthrachinonen.

**13.** Verfahren gemäß Anspruch 12, **dadurch gekennzeichnet, dass** die Oxidationsmittel an wasserunlöslichen inerten Polymeren festphasengebunden vorliegen und somit von den Reaktionsprodukten leicht abtrennbar und regenerierbar sind.

**14.** Verfahren nach Ansprüche 8 oder 11, **dadurch gekennzeichnet, dass** die reduktive oder oxidative Modifizierung der terminalen Aldehydfunktionen auf elektrochemischem Wege oder auf biochemischen Wege durch geeignete Enzyme oder Mikroorganismen erfolgt.

**15.** Verfahren nach irgendeinem der Ansprüche 1 bis 7, **dadurch gekennzeichnet, dass** die Modifizierung der terminalen Aldehydgruppen durch geeignete Kopplungs-Reagenzien erfolgt, die ausgewählt sind aus der Gruppe bestehend aus Blausäure, Natriumcyanid, Kaliumcyanid, Aminen, Aminosäuren, Peptiden und Proteinen.

**16.** Verfahren nach irgendeinem der Ansprüche 1 bis 15, **dadurch gekennzeichnet, dass** die Derivatisierung (ii) der Kohlenhydrate mittels Acylierungs-, Hydroxyalkylierungs- oder Carboxyalkylierungsreagenzien erfolgt.

**17.** Verfahren nach Anspruch 16, **dadurch gekennzeichnet, dass** die Hydroxyalkylierungsreagenzien ausgewählt sind aus 2-Chlorethanol und Epoxiden mit 2 bis 4 Kohlenstoffatomen.

**18.** Verfahren nach Anspruch 17, **dadurch gekennzeichnet, dass** die Epoxide ausgewählt sind aus der Gruppe bestehend aus Ethylenoxid, Propylenoxid und Hydroxymethyloxiran.

**19.** Verfahren nach Anspruch 16, **dadurch gekennzeichnet, dass** die Carboxyalkylierungsreagenzien ausgewählt sind aus der Gruppe bestehend aus α-Halogencarbonsäuren mit 2 bis 8, vorzugsweise 3 bis 6, besonders bevorzugt 4 oder 5 Kohlenstoffatomen und α-Halogendicarbonsäuren mit 3 bis 8, vorzugsweise 3 bis 6, besonders bevorzugt 3 oder 4 Kohlenstoffatomen.

**20.** Verfahren nach Anspruch 16, **dadurch gekennzeichnet, dass** die Carboxyalkylierungsreagenzien ausgewählt sind aus der Gruppe bestehend aus α,β-ungesättigten Carbonsäuren und Carbonsäureestern mit 3 bis 8, vorzugsweise 4 bis 7, besonders bevorzugt 5 oder 6 Kohlenstoffatomen.

**22.** Verfahren nach Anspruch 16, **dadurch gekennzeichnet, dass** die Acylierungsreagenzien ausgewählt sind aus der Gruppe bestehend aus:
(i) Mono- oder Dicarbonsäuren, mit 2 bis 8 vorzugsweise 3 bis 6, besonders bevorzugt 4 oder 5 Kohlenstoffatomen.
(ii) Carbonsäureanhydriden, die sich von aliphatischen Monocarbonsäuren mit 2 bis 8, vorzugsweise 3 bis 6, besonders bevorzugt 4 oder 5 Kohlenstoffatomen oder von aliphatischen Dicarbonsäuren mit 3 bis 8, vorzugsweise 4 bis 7, besonders bevorzugt 5 oder 6 Kohlenstoffatomen ableiten; und
(iii) Carbonsäurehalogeniden, ausgewählt aus Chloriden, Bromi den oder Iodiden, vorzugsweise Chloriden der unter (i) genannten Mono- oder Dicarbonsäuren.

**23.** Stärkederivate erhältlich nach dem Verfahren gemäß irgendeinem der Ansprüche 1 bis 22.

**24.** Stärkederivat gemäß Anspruch 23, **dadurch gekennzeichnet, dass** es sich um eine Hydroxyethylstärke handelt.

**25.** Stärkederivat gemäß Anspruch 23, **dadurch gekennzeichnet, dass** es sich um eine Carboxymethyl-hydroxyethylstärke handelt.

**26.** Stärkederivat gemäß Anspruch 23, **dadurch gekennzeichnet, dass** es sich um eine Carboxymethylstärke handelt.

**27.** Carboxymethylstärke gemäß Anspruch 26 mit
einem Molekulargewicht von 20.000 bis 1.000.000, vorzugsweise 40.000 bis 500.000, besonders bevorzugt 60.000 bis 200.000;
einem Verzweigungsgrad von 0 bis 6 %, vorzugsweise 2 bis 5 %, besonders bevorzugt 3 bis 5 %;
einem Substitutionsgrad MS im Bereich von 0,01 bis 1,00, vorzugsweise von 0,05 bis 0,60, besonders bevorzugt von 0,1 bis 0,45;
einem Substitutionsgrad DS im Bereich von 0,01 bis 1,00, vorzugsweise von 0,05 bis 0,60, besonders bevorzugt von 0,1 bis 0,45;
wobei wenigstens 70 %, vorzugsweise über 80 %, besonders bevorzugt über 85 % der Carboxymethylgruppen an den sekundären Hydroxylgruppen am C-Atom 2 und/oder 3 der Anhydroglucoseeinheiten lokalisiert sind.

**28.** Pharmazeutische Zusammensetzung umfassend wenigstens ein Stärkederivat gemäß irgendeinem der Ansprüche 23 bis 27.

**29.** Pharmazeutische Zusammensetzung gemäß Anspruch 28 umfassend wenigstens ein Stärkederivat in wässriger Lösung.

**30.** Pharmazeutische Zusammensetzung gemäß Anspruch 28 und 29, **dadurch gekennzeichnet, dass** sie einen physiologischen pH-Wert aufweist.

**31.** Pharmazeutische Zusammensetzung gemäß Anspruch 28 bis 30, **dadurch gekennzeichnet, dass** sie eine Osmolarität zwischen 250 und 400 mOsmol/l aufweist.

**32.** Pharmazeutische Zusammensetzung gemäß Anspruch 28 bis 31, **dadurch gekennzeichnet, dass** die wässrige Lösung zusätzlich einen Kationenanteil zwischen 50 und 170 mmol/l und einen Anionenanteil zwischen 50 und 170 mmol/l aufweist.

**33.** Verfahren zur Blutplasmasubstitution, **dadurch gekennzeichnet, dass** eine pharmazeutische Zusammensetzung gemäß irgendeinem der Ansprüche 28 bis 32 verwendet wird.

**34.** Verwendung eines Stärkederivates gemäß irgendeinem der Ansprüche 23 bis 27 zur Herstellung eines Arzneimittels zur Volumentherapie, zur Verbesserung der Mikrozirkulation, zur Hämodilution oder zum Blutvolumenersatz.
